# EUROPÄISCHE PATENTSCHRIFT

(11) **EP 0 491 001 B1**
(45) Veröffentlichungstag und Bekanntmachung des Hinweises auf die Patenterteilung: **01.12.1993**
(21) Anmeldenummer: 90914709.2
(22) Anmeldetag: 01.09.1990
(51) Int. Cl.: C07F 9/6561, C07F 9/6512, A61K 31/675

(54) **NEUE AMIDINGRUPPEN-HALTIGE DIPHOSPHONSÄUREDERIVATE, VERFAHREN ZU DEREN HERSTELLUNG UND DIESE VERBINDUNGEN ENTHALTENDE ARZNEIMITTEL**
NEW DIPHOSPHONIC ACID DERIVATIVES CONTAINING AMIDINE GROUPS, PROCESS FOR PREPARING THEM AND DRUGS CONTAINING THESE COMPOUNDS
NOUVEAUX DERIVES D'ACIDES DIPHOSPHONIQUES CONTENANT DES GROUPES AMIDINES, PROCEDE POUR LEUR FABRICATION ET MEDICAMENTS RENFERMANT CES COMPOSES

(30) Priorität: 09.09.1989 DE 3930130
(43) Veröffentlichungstag der Anmeldung: 24.06.1992
(73) Patentinhaber: BOEHRINGER MANNHEIM GMBH, 68298 Mannheim (DE)
(72) Erfinder: BOSIES, Elmar, D-6940 Weinheim (DE); ZILCH, Harald, D-6800 Mannheim 31 (DE)
(74) Vertreter: Weber, Manfred, Dr.
(86) Internationale Anmeldenummer: EP9001469
(87) Internationale Veröffentlichungsnummer: WO9103481

(56) Entgegenhaltungen:
- DE-A- 2 439 355
- DE-A- 3 719 513
- DE-A-36 409 38
- DE-B- 2 316 396
- US-A- 3 941 772
- US-A- 4 086 334

## Beschreibung

Die vorliegende Erfindung betrifft neue Amidingruppenhaltige Diphosphonsäurederivate, Verfahren zu deren Herstellung sowie Arzneimittel, die diese Substanzen enthalten.

In der DE 18 13 659 sind Diphosphonsäurederivate beschrieben, von denen die 1-Hydroxyethan-1,1-diphosphonsäure als Mittel zur Behandlung von Morbus Paget Bedeutung erlangt hat.

Das EP 282 320-A-1 beschreibt substituierte 3-Isoxazolyl-aminomethylendiphosphonsäuren und deren Ester mit antihypercalcä mischer und antiarthritischer Wirkung.

In der EP 282 309-A-2 sind "Azol"-aminomethylendiphosphonsäuren als Hypercalcämie-Inhibitoren beschrieben.

Weiterhin kennt man aus JP 63/150 290-A-2 Aminomethylendiphosphonsäuren als Regulatoren des Calcium-Metabolismus und aus EP 274 158-A-1 Tetrahydropyrimidinyl- und Tetrahydropyridylaminomethylendiphosphonsäuren zur Behandlung eines abnormen Calcium- und Phosphat-Stoffwechsels.

In den Patentschriften US 4 447 256-A, JP 55/89 210, JP 55/98 104, JP 55/98 105, JP 55/94 307 und JP 55/94 309 sind u.a. Pyridylaminomethylenphosphonsäuren als Herbizide genannt.

Amidingruppen-haltige geminale Diphosphonsäuren mit nur einem Stickstoff in dem Ring, an den die beiden Phosphonsäuren gebunden sind, kennt man aus der DE 3 208 600-A-1 und Liebigs Ann. Chem. 1982, 275.

Es wurde nun gefunden, daß analoge Derivate dieser Verbindungen mit einer Amidinstruktur im Ring ebenfalls sehr gute Calcium-Komplexbildner sind, daneben aber auch eine ausgezeichnete Wirkung auf den Calciumstoffwechsel zeigen und damit zur breiten Behandlung von Calciumstoffwechselstörungen geeignet sind. Sie lassen sich vor allem sehr gut dort einsetzen, wo der Knochenauf- und -abbau gestört ist, d.h. sie sind geeignet zur Behandlung von Erkrankungen des Skelettsystems wie z.B. Osteoporose, Morbus Paget, Morbus Bechterew u.a..

Aufgrund dieser Eigenschaften finden sie aber auch Verwendung in der Therapie von Knochenmetastasen, der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenarativen Arthrose.

Gegenstand der vorliegenden Erfindung sind demnach Diphosphonate der allgemeinen Formel I 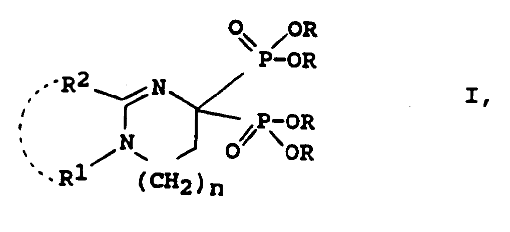
in der
- R: Wasserstoff oder C₁-C₄-Alkyl,
- R¹: Wasserstoff, C₁-C₆-Alkyl, Aryl, Aryl-C₁-C₄-alkyl, Amino-C₁-C₆-alkyl, C₁-C₆-Alkylamino-C₁-C₄-alkyl, C₁-C₆-Dialkylamino-C₁-C₄-alkyl, C₁-C₄-Alkoxy-C₁-C₄-alkyl, C₁-C₄-Alkylthio-C₁-C₄-alkyl, C₃-C₇-Alkenyl,
- R²: R¹ oder C₂-C₇-Alkenyl, C₁-C₆-Alkylmercapto, C₁-C₆-Alkoxy, Phenoxy-C₁-C₄-alkyl, Amino, C₁-C₄-Alkylamino, Di-C₁-C₄-alkylamino, Morpholino, Thiomorpholino, Pyrrolidino, Piperidino, Hexamethylenimino, Pyrazolino, Imidazolino,
- n: 0, 1 oder 2

sein kann,
und R¹ und R² gemeinsam mit dem Kohlenstoff- und dem Stickstoffatom an die sie gebunden sind, einen heterocyclischen Fünf-, Sechs- oder Siebenring mit 1-4 Heteroatomen bilden können, wobei die Heteroatome gleich oder verschieden sein können und Sauerstoff, Stickstoff oder Schwefel bedeuten und der annelierte Ring gegebenenfalls durch eine oder mehrere C₁-C₆-Alkyl-, C₁-C₆-Alkoxy-, C₁-C₆-Alkylmercaptogruppen, Hydroxy, Amino, Nitro, Halogen oder Halogenmethyl substituiert sein kann,
sowie deren pharmakologisch unbedenkliche Salze.

Für den Fall, daß R¹ und R² gemeinsam mit dem Kohlenstoff-und dem Stickstoffatom an die sie gebunden sind einen heterocyclischen Ring bilden, handelt es sich dabei bevorzugt um den annelierten Pyrrolin-, Pyrazolin-, Imidazolin-, Triazolin-, Oxazolin-, Isoxazolin-, Thiazolin-, Isothiazolin-, Thiadiazolin-, Oxadiazolin-, Pyridin-, Pyrimidin-, Pyrazin-, Pyridazin-, oxazin-, Thiazin-, Triazin-, Tetrazin-, Diazepin-, Azacycloheptan-, -hepten-, -heptadien-, oxaazacycloheptan-, -hepten-, -heptadien-, Thiaazacycloheptan-, -hepten- oder -heptadienring. Die annelierten Heterocyclen können hydriert sein oder bis zu zwei Doppelbindungen enthalten.

Alkyl-, Alkoxy-, Alkylamino- und Alkylmercapto-Substituenten in den Resten R¹ und R² können geradkettig oder verzweigt sein und 1-6, vorzugsweise 1-4 Kohlenstoffatome enthalten. Bevorzugt ist der Methyl-, Ethyl-, Isopropyl-, Methoxy-, Ethoxy-, Methylamino-, Ethylamino-, Methylmercapto- und Ethylmercaptorest.

Unter Aryl ist bevorzugt der Phenylrest zu verstehen. Halogen steht allgemein für Fluor, Chlor und Brom, vorzugsweise Fluor und Chlor.

R soll bevorzugt Wasserstoff darstellen.

Bevorzugter Alkenylrest ist der Allyl- oder Butenylrest, Di-alkylamino steht vorzugsweise für Dimethyl- und Diethylamino und die geradkettigen oder verzweigten Alkylenketten der Arylalkyl-, Phenoxyalkyl-, Alkoxyalkyl-, Alkylthioalkyl-, Alkylaminoalkylreste stellen bevorzugt die Methylen-, Ethylen-, Isopropylenkette dar. Die entsprechenden C₁-C₄-Alkylteile der angeführten Substituenten stehen vorzugsweise für Methyl und Ethyl.

Bei den monocyclischen Verbindungen der allg. Formel I handelt es sich bevorzugt um substituierte 5,6-Dihydro-1H-pyrimidin-4,4-diphosphonsäuren und 1,3-Diaza-2-cyclohepten-4,4-diphosphonsäuren der Formel I. Bevorzugte bicyclische Verbindungen der allgemeinen Formel I sind das Pyridopyrimidin-, Pyrimidopyrimidin-, Oxazolopyrimidin-, Thiazolopyrimidin-, Triazolopyrimidin-, Oxadiazolopyrimidin-, Thiadiazolopyrimidin-, Imidazopyrimidin-, Pyrimidothiazin-, Pyridodiazepin-, Oxazolodiazepin-, Thiazolo- diazepin- und Pyrrolopyrimidin-Ringsystem.

n ist vorzugsweise 1 oder 2.

Besonders bevorzugte Bicyclen sind die Pyrido-(1,2-a)-pyrimidin-, Oxazolo-(3,2-a)-pyrimidin-, Thiazolo-(3,2-a)-pyrimidin-, 1,2,4-Triazolo-(1,5-a)-pyrimidin-, Pyrimido-(1,2-a)-pyrimidin-, Pyrimido-(2,1-b)-1,3-thiazin- und Pyrimido-(1,2-a)-1,3- diazepindiphosphonsäuren.

Die Verbindungen können als Stereoisomerengemisch oder als reine cis- bzw. trans-Isomere vorliegen.

Asymmetrische Kohlenstoffatome können die R- bzw. S-Konfiguration besitzen, und die Verbindungen können optisch aktiv oder als Racemate vorliegen.

Die Verbindungen der allg. Formel I werden nach an sich bekannten Verfahren hergestellt, vorzugsweise indem man ein Pyrimidinon der allgemeinen Formel II 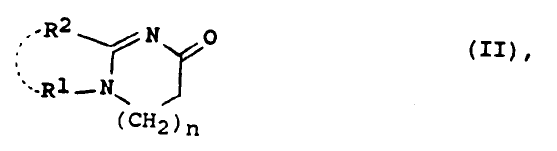
in der R¹, R² und n die oben angegebene Bedeutung haben, mit einem Gemisch aus phosphoriger Säure oder Phosphorsäure und einem Phosphorhalogenid oder phosphorylhalogenid umsetzt, oder das Phosphorhalogenid auch allein in Gegenwart von Wasser zur Reaktion bringen kann, und anschließend zur freien Diphosphonsäure hydrolysiert, oder gewünschtenfalls die isolierten Verbindungen der allgemeinen Formel I in deren Ester bzw. in pharmakologisch unbedenkliche Salze überführt.

Die bei dem Herstellungsverfahren eingesetzten Pyrimidinone der allg. Formel II werden mit 1-5, vorzugsweise 2-3 mol phosphoriger Säure oder Phosphorsäure und 1-5, vorzugsweise 2-3 mol Phosphorylhalogenid, phosphortrihalogenid oder Phosphorpentahalogenid versetzt und bei 80-130°C, vorzugsweise 100°C zur Reaktion gebracht. Bei den Phosphor- oder Phosphorylhalogeniden handelt es sich vorzugsweise um die Chloride bzw. Bromide. Man kann die Reaktion auch in Gegenwart von Verdünnungsmitteln wie Halogenkohlenwassarstoffen, insbesondere Chlorbenzol, Tetrachlorethan oder auch Dioxan, gegebenenfalls unter Zusatz von Wasser, durchführen. Die anschließende Hydrolyse erfolgt durch Erhitzen mit Wasser, zweckmäßiger jedoch mit halbkonzentrierter Salz- oder Bromwasserstoffsäure.

Die freien Diphosphonsäuren der allgemeinen Formel I können durch Erhitzen mit Orthoameisensäurealkylestern in die entsprechenden Tetraaalkylester überführt werden und zu Diestern oder wieder zu den freien Tetrasäuren verseift werden. Die Verseifung zu Diestern geschieht in der Regel dadurch, daß man den Tetraalkylester mit einem Alkalihalogenid, vorzugsweise Natriumjodid in einem geeigneten Lösungsmittel wie z.B. Aceton bei Zimmertemperatur behandelt.

Hierbei entsteht das symmetrische Diester/Dinatriumsalz, das gegebenenfalls durch einen sauren Ionenaustauscher in die Diester/ Disäure umgewandelt werden kann. Die Verseifung der Ester zu freien Diphosphonsäuren geschieht in der Regel durch Kochen mit Salz- oder Bromwasserstoffsäure. Man kann jedoch auch eine Spaltung mit Trimethylsilylhalogenid, vorzugsweise dem Bromid oder Jodid vornehmen.

Verbindungen der allgemeinen Formel I lassen sich auch nachträglich in andere Verbindungen der allgemeinen Formel I überführen. Dies betrifft z.B. die Hydrierung ungesättigter Substituenten zu den entsprechenden aliphatischen Resten oder die partielle bzw. vollständige Hydrierung von Doppelbindungen in mono- und bicyclischen Verbindungen.

Weiterhin lassen sich durch Abspaltung von Schutzgruppen Verbindungen der allgemeinen Formel I in andere Verbindungen der Formel I überführen.

Als pharmakologisch verträgliche Salze werden vor allem Mono- bzw. Dialkali- oder Ammoniumsalze verwendet, die in üblicher Weise z. B. durch Titration der Verbindungen mit anorganischen oder organischen Basen wie z.B. Natrium- oder Kaliumhydrogencarbonat, Natronlauge, Kalilauge, wässrigem Ammoniak oder Aminen wie z.B. Trimethyl-, Triethyl- oder Cyclohexylamin herstellt werden.

Die Salze werden in der Regel durch Umfällen aus Wasser/Methanol oder Wasser/Aceton gereinigt.

Die erfindungsgemäßen neuen Substanzen der Formel I und ihre Salze können in flüssiger oder fester Form enteral oder parenteral appliziert werden. Hierbei kommen alle üblichen Applikationsformen infrage, beispielsweise Tabletten, Kapseln, Dragees, Sirupe, Lösungen, Suspensionen etc.. Als Injektionsmedium kommt vorzugsweise Wasser zur Anwendung, welches die bei Injektionslösungen üblichen Zusätze wie Stabilisierungsmittel, Lösungsvermittler und Puffer enthält.

Derartige Zusätze sind z.B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten.

Die Dosierung kann von verschiedenen Faktoren, wie Applikationsweise, Spezies, Alter und/oder individuellem Zustand abhängen. Die täglich zu verabreichenden Dosen liegen bei etwa 1-1000 mg/Mensch, vorzugsweise bei 10-200 mg/Mensch und können auf einmal oder mehrere Male verteilt eingenommen werden.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden Diphosphonsäuren, sowie deren Natriumsalze, Methyl- bzw. Ethylester:
5,6-Dihydro-1H-pyrimidin-4,4-diphosphonsäure
1-Methyl-5,6-dihydro-1H-pyrimidin-4,4-diphosphonsäure
2-Dimethylamino-1-methyl-5,6-dihydro-1H-pyrimidin-4,4-di-phosphonsäure
2-Phenyl-5,6-dihydro-1H-pyrimidin-4,4-diphosphonsäure
1,2-Dimethyl-5,6-dihydro-1H-pyrimidin-4,4-diphosphonsäure
2-Ethoxy-1-methyl-5,6-dihydro-1H-pyrimidin-4,4-diphosphonsäure
2-Ethylmercapto-1-methyl-5,6-dihydro-1H-pyrimidin-4,4-di-phosphonsäure
1,2-Dimethyl-l,3-diaza-2-cyclohepten-4,4-diphosphonsäure
2-Methyl-1,3-diaza-2-cyclohepten-4,4-diphosphonsäure
2-Amino-1,3-diaza-2-cyclohepten-4,4-diphosphonsäure
2-Ethoxy-1-methyl-1,3-diaza-2-cyclohepten-4,4-diphosphonsäure
2-Ethylmercapto-1-methyl-1,3-diaza-2-cyclohepten-4,4-di-phosphonsäure
2,3,4,5-Tetrahydropyrido-(1,2-a)-1,3-diazepin-2,2-di-phosphonsäure
2,3,4,5,7,8,9,10-Octahydropyrido-(1,2-a)-1,3-diazepin-2,2-diphosphonsäure
8-Chlor-2,3,4,5,7,8,9,10-octahydropyrido-(1,2-a)-1,3-diazepin-2,2-diphosphonsäure
3,4,6,7-Tetrahydro-2H,8H-pyrimido-(2,1-b)-1,3-thiazin-8,8-diphosphonsäure
3-Methyl-5,6-dihydro-7H-thiazolo-(3,2-a)-pyrimidin-7,7-diphosphonsäure
3-Methyl-2,3,5,6-tetrahydro-7H-thiazolo-(3,2-a)-pyrimidin-7,7-diphosphonsäure
3-Methyl-2,3-5,6-tetrahydro-7H-oxazolo-(3,2-a)-pyrimidin-7,7- diphosphonsäure
7-Chlor-3,4-dihydro-2H-pyrido-(1,2-a)-pyrimidin-2,2-di-phosphonsäure
7-Methyl-3,4,5,6,7,8,9-hexahydro-2H-pyrido-(1,2-a)-pyrimidin-2,2- diphosphonsäure
3,4,7,8-Tetrahydro-6H-pyrrolo-(1,2-a)-pyrimidin-2,2-di-phosphonsäure
2,3,5,6-Tetrahydro-1H-imidazo-(1,2-a)-pyrimidin-7,7-di-phosphonsäure
3,4,6,7,8,9-Hexahydro-2H-pyrimido-(1,2-a)-pyrimidin-2,2-diphosphonsäure
6,7-Dihydro-1,2,4-triazolo-(1H)-(1,5-a)-pyrimidin-5,5-di-phosphonsäure

Die nachfolgenden Beispiele zeigen einige der Verfahrensvarianten, die zur Synthese der erfindungsgemäßen Verbindungen verwendet werden können. Sie sollen jedoch nicht eine Einschränkung des Erfindungsgegenstandes darstellen. Die Verbindungen fallen in der Regel als hochschmelzende Festprodukte an (Mono- oder Dinatriumsalz), deren Struktur durch ¹H-, ³¹P- und gegebenenfalls durch 13C-NMR-Spektroskopie gesichert wurde. Die Reinheit der Substanzen wurde mittels C,H,N,P,S, Na-Analyse sowie durch Dünnschichtelektrophorese (Cellulose, Oxalat-Puffer von pH = 4.0) bestimmt. Zur Charakterisierung der einzelnen Verbindungen werden die Mᵣₑₗ-Werte (= relative Mobilität) bezogen auf Pyrophosphat (Mᵣₑₗ = 1) angegeben.

Die als Ausgangsverbindungen eingesetzten Pyrimidinone der allgemeinen Formel II werden nach bekannten Verfahren hergestellt, z.B. indem man substituierte acyclische oder cyclische Amidine bzw. Guanidine mit Acrylsäureestern, Acrylsäurehalogeniden oder 3-Brompropionsäurehalogeniden zur Reaktion bringt.

Die für die Beispiele benötigten Edukte wurden, sofern sie nicht kommerziell erhältlich waren, in Analogie zu folgenden Literaturstellen synthetisiert: Liebigs Ann. Chem. 1974, 593; Chem. Ber. 104, 3961 (1971)); Chem. Pharm. Bull. 19, 764 (1971); Chem.

Pharm. Bull. 20, 901 (1972); Monatsh. Chem. 116, 237 (1985).

### Beispiel 1

### 3,4-Dihydro-2H-pyrido-(1,2-a)-pyrimidin-2,2-diphosphonsäure

5g 3,4-Dihydro-pyrido-(1,2-a)-pyrimidin-2-on (Fluka) wurden bei 80° C mit 5.5 g H₃PO₃ verschmolzen und unter Rühren mit 5.9 ml PCl₃ versetzt. Nach 20 Stunden bei der angegebenen Temperatur wurde mit 70 ml 2 N HCl versetzt, 8 Stunden bei 100°C gerührt und nach dem Abkühlen vom ungelösten Anteil abf iltriert. Das Filtrat wurde im Rotationsverdampfer eingeengt und durch Ionenaustauscherchromatographie an Amberlite IR 120 (H⁺-Form) mit Wasser als Eluens gereinigt. Die nach Elektrophorese einheitlichen Fraktionen wurden im Vakuum eingedampft und das zurückbleibende Öl nach Lösen in wenig Wasser durch Zugabe von Aceton zur Kristallisation gebracht. Mᵣₑₗ. 0.33, Ausb. 3.8 g (38 % d.Th.), Schmp. 300-304°C.

### Beispiel 2

### 3,4,6,7,8,9-Hexahydro-2H-pyrido-(1,2-a)-pyrimidin-2,2-diphosphonsäure 3 g der in Beispiel 1 erhaltenen Diphosphonsaure wurden in 250 ml Wasser gelöst und nach Zugabe von 1.5 g PtO₂ bei Atmosphärendruck bis zur Aufnahme der berechneten Wasserstoffmenge hydriert. Nach Abtrennen des Katalysators und Einengen im Vakuum wurde der Rückstand aus Wasser/Aceton zur Kristallisation gebracht. Mᵣₑₗ. 0.33, Ausb. 2.61 g (85 % d.Th.), Schmp. 279-285°C (Zers.).

### Beispiel 3

### 2,3,5,6-Tetrahydro-7H-oxazolo-(3,2-a)-pyrimidin-7,7-di-phosphonsäure

5.7 g 2,3,5,6-Tetrahydro-7H-oxazolo-(3,2-a)-pyrimidin-7-on (Schmp. 146-147°C) wurden bei 60°C mit 6.7 g H₃PO₃ verschmolzen, unter Rühren langsam mit 7.5 ml POCl₃ versetzt und 36 Stunden bei 60°C gehalten. Dann wurde das überschüssige POCl₃ im Vakuum abgezogen, der Rückstand mit 80 ml Wasser versetzt und die klare Lösung für 1 Stunde auf 100°C erhitzt. Nach dem Abkühlen wurde wie bei Bsp. 1 beschrieben, aufgearbeitet und gereinigt.
Mᵣₑₗ. 0.42, Ausb. 1.4 g (12 % d.Th.), Schmp. 145°C (Zers.).

### Beispiel 4

### 6,7-Dihydro((1H)-1,2,4-triazolo-(1,5-a)-pyrimidin-5,5-di-phosphonsäure

2 g 6,7-Dihydro-(1H)-1,2,4-triazolo-(1,5-a) -pyrimidin-5-on (Schmp. 237-239°C) wurden analog zu Bsp. 3 8 Stunden bei 80°C zur Reaktion gebracht und 3 Stunden bei 100°C hydrolysiert. Die Aufarbeitung und Reinigung erfolgte ebenfalls in Anlehnung an die Bsp. 1 und 3. Mᵣₑₗ. 0.39, Ausb. 0.95 g (23 % d.Th.), Schmp. 167-171°C (Zers.).

### Beispiel 5

### 3,4-Dihydro-2H-pyrimido-(1,2-a)-pyrimidin-2,2-diphosphonsäure

In Analogie zu Bsp. 4 wurden 1.5 g 3,4-Dihydro-2H-pyrimido-(1,2- a)-pyrimidin-2-on (Schmp. 263°C) umgesetzt.
Mᵣₑₗ. 0.6, Ausb. 0.82 g (28 % d.Th.), Schmp. >85°C (Zers.).

### Beispiel 6

### 2-Methyl-5,6-dihydro-1H-pyrimidin-4,4-diphosphonsäure

5 g 2-Methyl-5,6-dihydro-1H-pyrimidin-4-on (Schmp. 123 -125°C) wurden in Anlehnung an die Bsp. 1, 3 und 4 umgesetzt.
Mᵣₑₗ. 0.31, Ausb. 1,28 g (14 %d.Th.), Schmp. >101°C (Zers.).

### Beispiel 7

### 2-Amino-1-methyl-5,6-dihydro-1H-pyrimidin-4,4-diphosphonsäure

1.2 g 2-Amino-1-methyl-5,6-dihydro-1H- pyrimidin-4-on (Schmp. 255-257°C) wurden in Anlehnung an die Bsp. 1, 3, 4 und 6 umgesetzt. Mᵣₑₗ. 0.32, Ausbeute 0.6 g (23 % d. Th.), Schmp. 308°C.

## Patentansprüche

1. Verbindungen der Formel I 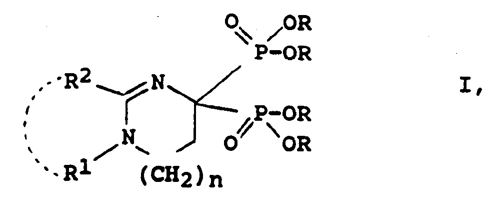 in der
R Wasserstoff oder C₁-C₄-Alkyl,
R¹ Wasserstoff, C₁-C₆-Alkyl, Aryl, Aryl-C₁-C₄-alkyl, Amino-C₁-C₆-alkyl, C₁-C₆-Alkylamino-C₁-C₄-alkyl, C₁-C₆-Dialkylamino-C₁-C₄ -alkyl, C₁-C₄-Alkoxy-C₁-C₄-alkyl, C₁-c₄- Alkylthio-C₁-C₄-alkyl, C₃-C₇-Alkenyl,
R² R¹ oder C₂-C₇-Alkenyl, C₁-C₆-Alkylmercapto, C₁-C₆-Alkoxy, Phenoxy-C₁-C₄-alkyl, Amino, C₁-C₄-Alkylamino, Di-C₁-C₄-alkylamino, Morpholino, Thiomorpholino, Pyrrolidino, Piperidino, Hexamethylenimino, Pyrazolino, Imidazolino,
n 0, 1 oder 2
sein kann,
und R¹ und R² gemeinsam mit dem Kohlenstoff- und dem Stickstoffatom an die sie gebunden sind, einen heterocyclischen Fünf-, Sechs- oder Siebenring mit 1-4 Heteroatomen bilden können, wobei die Heteroatome gleich oder verschieden sein können und Sauerstoff, Stickstoff oder Schwefel bedeuten und der annelierte Ring gegebenenfalls durch eine oder mehrere C₁-C₆-Alkyl-, C₁-C₆-Alkoxy-, C₁-C₆-Alkylmercaptogruppen, Hydroxy, Amino, Nitro, Halogen oder Halogenmethyl substituiert sein kann,
sowie deren pharmakologisch unbedenkliche Salze.

2. Verfahren zur Herstellung von Verbindungen Formel I 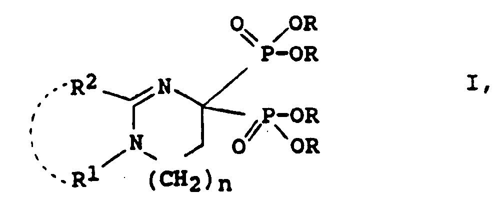 in der
R Wasserstoff oder C₁-C₄-Alkyl,
R¹ Wasserstoff, C₁-C₆-Alkyl, Aryl, Aryl-C₁-C₄-alkyl, Amino-C₁-C₆-alkyl, C₁-C₆-Alkylaminol-C₁-C₄-alkyl, C₁-C₆-Dialkylamino-C₁-C₄-alkyl, C₁-C₄-Alkoxy-C₁-C₄-alkyl, C₁-C₄-Alkylthio-C₁-C₄-alkyl, C₃-C₇-Alkenyl,
R² R¹ oder C₂-C₇-Alkenyl, C₁-C₆-Alkylmercapto, C₁-C₆-Alkoxy, Phenoxy-C₁-C₄-alkyl, Amino, C₁-C₄-Alkylamino, Di-C₁-C₄-alkylamino, Morpholino, Thiomorpholino, Pyrrolidino, Piperidino, Hexamethylenimino, Pyrazolino, Imidazolino,
n 0, 1 oder 2
sein kann,
und R¹ und R² gemeinsam mit dem Kohlenstoff- und dem Stickstoffatom an die sie gebunden sind, einen heterocyclischen Fünf-, Sechs- oder Siebenring mit 1-4 Heteroatomen bilden können, wobei die Heteroatome gleich oder verschieden sein können und Sauerstoff, Stickstoff oder Schwefel bedeuten und der annelierte Ring gegebenenfalls durch eine oder mehrere C₁-C₆-Alkyl-, C₁-C₆-Alkoxy-, C₁-C₆-Alkylmercaptogruppen, Hydroxy, Amino, Nitro, Halogen oder Halogenmethyl substituiert sein kann,
sowie deren pharmakologisch unbedenkliche Salze,
dadurch gekennzeichnet, daß man in an sich bekannter Weise eine Verbindung der Formel II 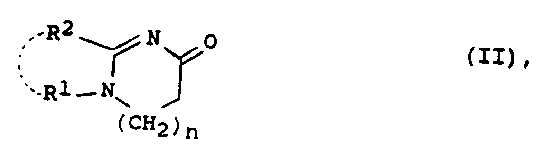 in der R¹, R² und n die oben angegebene Bedeutung haben, mit einem Gemisch aus phosphoriger Säure oder Phosphorsäure und einem Phosphorhalogenid oder Phosphorylhalogenid umsetzt, oder das Phosphorhalogenid auch allein in Gegenwart von Wasser zur Reaktion bringen kann, und anschließend zur freien Diphosphonsäure hydrolysiert, oder gewünschtenfalls die isolierten Verbindungen der allgemeinen Formel I in deren Ester bzw. in pharmakologisch unbedenkliche Salze überführt.

3. Arzneimittel, enthaltend eine Verbindung gemäß Anspruch 1 neben üblichen Träger- und Hilfsstoffen.

4. Verwendung von Verbindungen gemäß Anspruch 1 zur Herstellung von Arzneimitteln zur Behandlung von Calciumstoffwechselstörungen.

## Claims

1. Compounds of the formula I 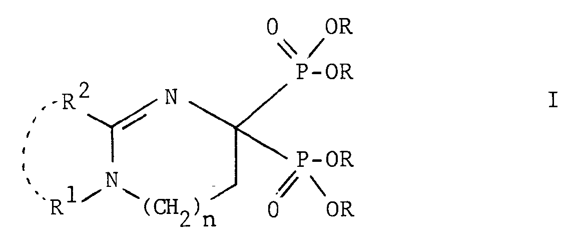 in which R can be hydrogen or C₁-C₄-alkyl, R¹ can be hydrogen, C₁-C₆-alkyl, aryl, aryl-C₁-C₄-alkyl, amino- C₁-C₆-alkyl,C₁-C₆-alkylamino-C₁-C₄-alkyl, C₁-C₆- dialkylamino-C₁-C₄-alkyl,C₁-C₄-alkoxy-C₁-C₄-alkyl, C₁-C₄-alkylthio-C₁-C₄-alkyl, C₃-C₇-alkenyl, R² can be R¹ or C₂-C₇-alkenyl, C₁-C₆-alkylmercapto, C₁-C₆-alkoxy, phenoxy-C₁-C₄-alkyl, amino, C₁-C₄-alkylamino, di-C₁-C₄- alkylamino, morpholino, thiomorpholino, pyrrolidino, piperidino, hexamethyleneimino, pyrazolino, imidazolino, n can be 0, 1 or 2 and R¹ and R², together with the carbon and the nitrogen atom to which they are attached, can form a heterocyclic five-, six- or seven-membered ring with 1 - 4 heteroatoms, whereby the heteroatoms can be the same or different and signify oxygen, nitrogen or sulphur and the annelated ring can possibly be substituted by one or more C₁-C₆-alkyl, C₁-C₆-alkoxy, C₁-C₆-alkylmercapto groups, hydroxyl, amino, nitro, halogen or halomethyl, as well as their pharmacologically acceptable salts.

2. Process for the preparation of compounds of the formula I 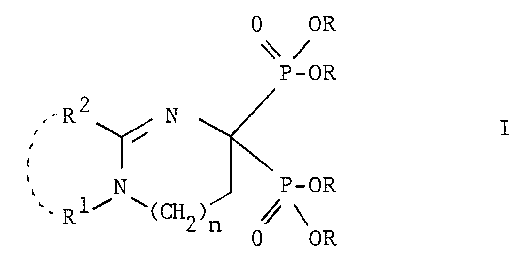 in which R can be hydrogen or C₁-C₄-alkyl, R¹ can be hydrogen, C₁-C₆-alkyl, aryl, aryl-C₁-C₄-alkyl, amino- C₁-C₆-alkyl, C₁-C₆-alkylamino-C₁-C₄-alkyl, C₁-C₆- dialkylamino-C₁-C₄-alkyl, C₁-C₄-alkoxy-C₁-C₄-alkyl, C₁-C₄-alkylthio-C₁-C₄-alkyl, C₃-C₇-alkenyl, R² can be R¹ or C₂-C₇-alkenyl, C₁-C₆-alkylmercapto, C₁-C₆- alkoxy, phenoxy-C₁-C₄-alkyl, amino, C₁-C₄- alkylamino, di-C₁-C₄-alkylamino, morpholino, thiomorpholino, pyrrolidino, piperidino, hexamethyleneimino, pyrazolino, imidazolino, n can be 0, 1 or 2 and R¹ and R², together with the carbon and the nitrogen atom to which they are bound, can form a heterocyclic five-, six- or seven-membered ring with 1 - 4 heteroatoms, whereby the heteroatoms can be the same or different and signify oxygen, nitrogen or sulphur and the annelated ring can possibly be substituted by one or more C₁-C₆-alkyl, C₁-C₆-alkoxy, C₁-C₆-alkylmercapto groups, hydroxyl, amino, nitro, halogen or halomethyl, as well as of their pharmacologically acceptable salts, characterised in that, in per se known manner, one reacts a compound of the formula II 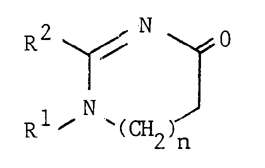 in which R¹, R² and n have the above-given meaning, with a mixture of phosphorous acid or phosphoric acid and a phosphorus halide or phosphoryl halide or one can also bring the phosphorus halide alone to reaction in the presence of water and subsequently hydrolyses to the free diphosphonic acid or, if desired, converts the isolated compounds of the general formula I into their esters or into pharmacologically acceptable salts.

3. Medicament containing a compound according to claim 1, besides usual carrier and adjuvant materials.

4. Use of compounds according to claim 1 for the preparation of medicaments for the treatment of calcium metabolism disturbances.

## Revendications

1. Composés de formule I 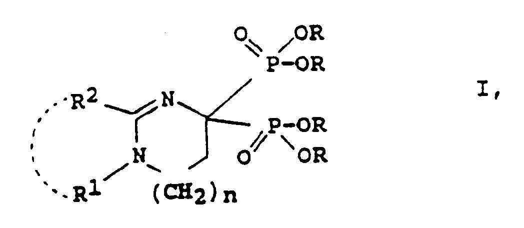 dans laquelle
R représente un atome d'hydrogène ou un groupe alkyl(C₁-C₄),
R¹ représente un atome d'hydrogène, un groupe alkyl(C₁-C₆), aryle, aryl-alkyl(C₁-C₄), amino-alkyl(C₁-C₆), alkyl(C₁-C₆)-amino-alkyl(C₁-C₄), dialkyl(C₁-C₆)-amino-alkyl(C₁-C₄), alcoxy(C₁-C₄)-alkyl(C₁-C₄), alkylthio(C₁-C₄)-alkyl(C₁-C₄), alcényl(C₃-C₇),
R² représente R¹ ou un groupe alcényl(C₂ - C₇), alkyl(C₁-C₆)mercapto, alcoxy(C₁-C₆), phénoxyalkyl(C₁-C₄), amino, alkyl(C₁-C₄)amino, dialkyl(C₁-C₄)amino, morpholino, thiomorpholino, pyrrolidino, pipéridino, hexaméthylèneimino, pyrazolino, imidazolino,
n vaut 0, 1 ou 2,
et R¹ et R² pouvant former, conjointement avec l'atome de carbone et l'atome d'azote auxquels ils sont liés, un hétérocycle à cinq, six ou sept maillons, comportant 1-4 hétéroatomes, les hétéroatomes pouvant être identiques ou différents, et représentent un atome d'oxygène, d'azote ou de soufre, et le cycle annellé pouvant être substitué éventuellement par un ou plusieurs groupes alkyl(C₁-C₆), alcoxy(C₁-C₆), alkyl(C₁-C₆)mercapto, hydroxy, amino, nitro, halogène ou halogénométhyle,
ainsi que leurs sels pharmacologiquement acceptables.

2. Procédé de préparation de composés de formule I 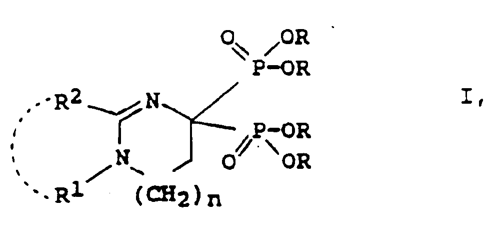 dans laquelle
R représente un atome d'hydrogène ou un groupe alkyl(C₁-C₄),
R¹ représente un atome d'hydrogène, un groupe alkyl(C₁-C₆), aryle, aryl-alkyl(C₁-C₄), amino-alkyl(C₁-C₆), alkyl(C₁-C₆)-amino-alkyl(C₁-C₄), dialkyl(C₁-C₆)-amino-alkyl(C₁-C₄), alcoxy(C₁-C₄)-alkyl(C₁-C₄), alkylthio(C₁-C₄)-alkyl(C₁-C₄), alcényl(C₃-C₇),
R² représente R¹ ou un groupe alcényl(C₂-C₇), alkyl(C₁-C₆)mercapto, alcoxy(C₁-C₆), phénoxyalkyl(C₁-C₄), amino, alkyl(C₁-C₄)amino, dialkyl(C₁-C₄)amino, morpholino, thiomorpholino, pyrrolidino, pipéridino, hexaméthylèneimino, pyrazolino, imidazolino,
n vaut 0, 1 ou 2,
et R¹ et R² pouvant former, conjointement avec l'atome de carbone et l'atome d'azote auxquels ils sont liés, un hétérocycle à cinq, six ou sept maillons, comportant 1-4 hétéroatomes, les hétéroatomes pouvant être identiques ou différents, et représentent un atome d'oxygène, d'azote ou de soufre, et le cycle annellé pouvant être substitué éventuellemen.t par un ou plusieurs groupes alkyl(C₁-C₆), alcoxy(C₁-C₆), alkyl(C₁-C₆)mercapto, hydroxy, amino, nitro, halogène ou halogénométhyle,
ainsi que leurs sels pharmacologiquement acceptables,
caractérisé en ce que de manière connue en soi, on fait réagir un composé de formule II 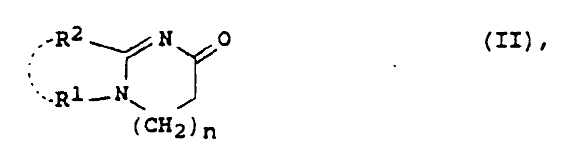 dans laquelle R¹, R² et n ont les significations mentionnées ci-dessus, avec un mélange d'acide phosphoreux et d'acide phosphorique, et un halogénure de phosphore ou un halogénure de phosphoryle, ou l'on faire fait réagir l'halogénure de phosphore même tout seul en présence d'eau, hydrolyse en acide diphosphonique libre, ou éventuellement, on transforme le composé de formule générale I isolé en son ester ou sel pharmacologiquement acceptable.

3. Médicament, contenant un composé selon la revendication 1, en plus des véhicules et adjuvants usuels.

4. Utilisation des composés selon la revendication 1, pour la préparation de médicaments destinés au traitement des troubles du métabolisme calcique.
